# EUROPEAN PATENT APPLICATION

(11) **EP 0 704 223 A1**
(43) Date of publication of application: **03.04.1996**
(21) Application number: 95918183.5
(22) Date of filing: 12.05.1995
(51) Int. Cl.: A61M 1/14, A61M 1/36, A61L 33/00

(54) **METHOD OF INHIBITING BLOOD COAGULATION DURING EXTRACORPOREAL BLOOD CIRCULATION AND DEVICE FOR RELEASING ANTITHROMBOTIC DRUG USED THEREIN**

(30) Priority: 17.05.1994 JP 102882/94
(71) Applicant: OTSUKA PHARMACEUTICAL FACTORY, INC., Naruto-shi Tokushima 772 (JP); OTSUKA PHARMACEUTICAL CO., LTD., Chiyoda-ku Tokyo 101 (JP)
(72) Inventor: IGUCHI, Seiichiro, Naruto-shi Tokushima 772 (JP); HIGASHINO, Rika, Itano-gun Tokushima 771-02 (JP); YAMATO, Minoru, Itano-gun Tokushima 771-12 (JP); YAMAMOTO, Hiroaki, Naruto-shi Tokushima 771-03 (JP); INAI, Masatoshi, Itano-gun Tokushima 771-15 (JP)
(74) Representative: Hansen, Bernd, Dr. Dipl.-Chem.
(86) International application number: JP9500917
(87) International publication number: WO9531228

(57) **Abstract**

A method of inhibiting the coagulation of blood during the extracorporeal circulation thereof by bringing a member for releasing an antithrombotic drug, prepared by dispersing a difficulty water-soluble antithrombotic drug in a macromolecular material, into contact with the circulating blood. A device for releasing an antithrombotic drug to be used in the above method comprises either the drug releasing member contained in a vessel provided with blood inlet and outlet ports at the respective ends thereof or the drug releasing member constituting at least the inner wall surface of the vessel. As the antithrombotic agent can be released sustainedly from the drug releasing member, the blood coagulation in the blood circuit can be inhibited as a result of the contact of the blood with the member during the extracorporeal circulation.

## Description

### TECHNICAL FIELD

The present invention relates to a method for anticoagulation of blood in extracorporeal circulation of blood, and an antithrombotic drug releasing device to be used for the same.

### BACKGROUND ART

In extracorporeal circulation represented by hemodialysis, heparin is mainly used as an anticoagulant. However, heparin has the problem that it causes side effects such as induction of allergic reactions (e.g. urticaria, asthmatic attack, fever, etc.), increase in neutral fat and free fatty acid in blood due to acceleration of secretion of lipoprotein lipase from vascular endothelial cells, acceleration of osteoporosis, decrease in platelet count and increase in platelet secretion material due to activation of platelet and the like. There also is the danger that heparin promotes the bleeding tendency to a patient having a hemorrhagic diathesis.

Further, heparin also has the problem that it bonds with antithrombin III (AT III) to exhibit an anticoagulation action so that sufficient effect can not be obtained in a patient wherein antithrombin III is decreased congenitally or in the course of the patient's lifetime.

On the other hand, protease inhibitors such as futhan, FOY, etc. are used for a patient having a hemorrhagic diathesis, however, their use is limited within a short period of time because metabolites are guanidine derivatives having a strong biotoxicity and they have a cumulative action. Further, they are strongly absorbed in a certain dialysis membrane (e.g. PAN membrane, AN-69 membrane, etc.) and require a high dose so as to obtain the effect. There also is the problem that they cause a desorption phenomenon from active carbon.

Low molecular weight heparin is used in place of heparin. Thereby, the problem about heparin is alleviated but is not completely eliminated.

Since all of the above drugs inhibit blood coagulation by the inhibition of blood coagulation factors, no consideration is given to the activation of platelet at the time of the extracorporeal circulation, thereby causing deterioration of dialysis efficiency due to fibrin-platelet deposition onto the dialysis membrane, decrease in platelet count and increase in platelet secretion material in a blood circuit and the like.

To the contrary, there is proposed a method using an antiplatelet agent as an anticoagulant for dialysis. That is, prostaglandin having a high water-solubility and its analogue are used as a trial but it requires a high dose so as to inhibit blood coagulation in a blood circuit. Therefore, the side effect such as blood pressure reduction (hypotention) is strong and the chemical stability is poor so that it is difficult to use them alone.

On the other hand, since antiplatelet agents such as ticlopidine, cilostazol, etc. are slightly soluble in water, it is difficult to administer them continuously into a blood circuit as an injection like heparin so that they were forced to be orally administered, thereby causing the following problems:
(1) It is necessary to administer from several hours to several days before the initiation of dialysis; and
(2) It requires a high dose so as to increase the concentration of the drug in the blood circuit because of systemic administration, thereby causing side effects.

Thus, they merely attain auxiliary alleviation of thrombus due to low dose, and are not suitable for practical application.

It is a main object of the present invention to solve the above problems, thereby providing a method for inhibiting blood coagulation at the time of the extracorporeal circulation using an antithrombotic drug which is slightly soluble in water, and an antithrombotic drug releasing device to be used for the same.

### DISCLOSURE OF THE INVENTION

In order to solve the above problems, the present inventors have studied intensively about a method for applying antithrombotic drugs, e.g. antiplatelet agents which are slightly soluble in water, such as cilostazol to the extracorporeal circulation. As a result, the following fact has been found, thus the present invention has been accomplished. That is, it is possible to control the release of the antithrombotic drug from the antithrombotic drug releasing member, which is formed by dispersing the antithrombotic drug in a certain polymer material, depending upon the kind of polymer material to be used; kind, amount and blending method of the antithrombotic drug; formulation of additives into the polymer material; and the like. Therefore, blood coagulation in the blood circuit can be inhibited effectively by bringing the antithrombotic drug releasing member into contact with blood, directly, at the blood circuit to continuously release the drug with a sufficient concentration in blood.

Accordingly, the method for anticoagulation of the present invention is characterized by bringing an antithrombotic drug releasing member, which is formed by dispersing an antithrombotic drug in a polymer material, into contact with blood at the time of the extracorporeal circulation of blood, said antithrombotic drug being slightly soluble in water.

The antithrombotic drug releasing member may be used at any section of the blood circuit for extracorporeal circulation, but it is preferred to use it at a section to take blood outside the body according to the relation with the concentration of the drug, which is close to a human body, i.e. a section to initiate the extracorporeal circulation. Further, the antithrombotic drug releasing member can also be used in one or more places in an extracorporeal circulation system.

The antithromogenic drug releasing device to be used in the method of the present invention is installed in the blood circuit for extracorporeal circulation, and is characterized by comprising a vessel provided with a blood inlet and a blood outlet and an antithrombotic drug releasing member contained in the vessel, said antithrombotic drug releasing member being formed by dispersing an antithrombotic drug in a polymer material.

Further, another antithromogenic drug releasing device in the present invention is characterized in that, an inner wall surface of a vessel provided with a blood inlet and a blood outlet is composed of an antithrombotic drug releasing member wherein an antithrombotic drug is dispersed in a polymer material. In this case, the vessel constitutes a part of a blood circuit.

### BRIEF EXPLANATION OF THE DRAWINGS

Fig. **1** is an explanatory view illustrating one embodiment of the method for anticoagulation of the present invention applied to an extracorporeal circulation system for hemodialysis.

Fig. **2** is an explanatory view illustrating the test device for evaluating dissolution properties of an antithrombotic drug in Example 1, schematically.

Fig. **3** is a plane view illustrating the vessel used in Fig. **2**.

Fig. **4** is a sectional view illustrating the vessel containing an antithrombotic drug releasing member used in Example 4.

Fig. **5** is an explanatory view illustrating the hemodialysis model used in Example 4, schematically.

Fig. **6** is a graph illustrating the results of a dissolution test using the hemodialysis model used in Example 4.

Fig. **7** is a sectional view illustrating a vessel for connecting with the blood circuit of Example 5.

Fig. **8** is a partially exploded perspective view illustrating a state where an antithrombotic drug releasing member is contained in the vessel of Fig. **7**.

Fig. **9** is a longitudinal sectional view illustrating the vessel for connecting with the blood circuit for artificial heart-lung prepared in Example 6.

Fig. **10** is a transverse sectional view illustrating the vessel shown in Fig. **9**.

### BEST MODE FOR CARRYING OUT THE INVENTION

The antithrombotic drug releasing member is obtained by uniformly dispersing one or more sorts of antithrombotic drugs in polymer materials such as polyvinyl chloride, ethylene-vinyl alcohol copolymer, polyacrylate, polymethacrylate, polycarbonate, acetylated cellulose, polyacrylonitrile, polyethylene terephthalate, polyamide, etc., and it can release the antithrombotic drug, continuously.

In the present invention, it is particularly preferred to use polyvinyl chloride, ethylene-vinyl alcohol copolymer, polymethacrylate, polycarbonate or acetylated cellulose, among the above polymer materials. These polymer materials may be used as they are as a main constituent material of the antithrombotic drug releasing member, or they may be used after applying on or permeating into the other material (e.g. woven fabric, synthetic resin foam, etc.). Further, these polymer materials may be used alone or in combination thereof.

The polymer material has hitherto been used as the material which directly contacts with blood, and it is proved that the polymer material has extremely high safety and stability.

The polymer material can be anyone which is suitable as the antithrombotic drug releasing material, and those having various physical properties can be employed. For example, in case of polyvinyl chloride, those having an average degree of polymerization of 800 to 8000, preferably 800 to 4500 are suitable. When using it as plasticized polyvinyl chloride, there can be added plasticizers such as di-2-ethylhexyl phthalate, di-n-decyl phthalate, tri-2-ethylhexyl trimellitate, etc., various stabilizers, secondary plasticizers, lubricants and the like.

In case of the ethylene-vinyl alcohol copolymer, the composition ratio of ethylene to vinyl alcohol can be appropriately varied according to the usage of the antithrombotic drug releasing member and processing method. Normally, it is preferred that the etylene content is 10 to 80 mol %. When the ethylene content exceeds 80 mol %, the blood compatibility and dispersibility of the antithrombotic drug becomes inferior. On the other hand, when the content is smaller than 10 mol %, the mechanical strength, the water resistance and molding adaptability in a melting method described hereinafter are deteriorated.

It is preferred to use methyl polymethacrylate as the polymethacrylate in view of facility of molding in the melting method described hereinafter.

It is preferred to use a polymer having a chemical structure of carbonate of bisphenol A as the polycarbonate.

As the acetylated cellulose, there can be used any one having any acetylation degree, but it is preferred to use triacetyl cellulose because of its high mechanical strength.

Examples of the antithrombotic drug contained in these antithrombotic drug releasing member include antiplatelet agents, thrombin inhibitor and the like. These antithrombotic drugs can be used alone or in combination thereof.

Examples of the antiplatelet agent include aspirin, indomethacin, sulfinpyrazone, 2-[4,5-bis(4-methoxyphenyl)thiazol-2-yl]pyrrole-1-acetic acid ethyl ester, 2-methyl-3-(1,4,5,6-tetrahydronicotinoyl)pyrazolo[1,5-a]pyridine, satigrel, d-indobufen, dipyridamol, cilostazol, 1-(cyclohexylmethyl)-4-[4-(2,3-dihydro-2-oxo-1H-imidazo [4,5-b]quinolin-7-yloxy)-1-oxobutyl]piperazine, 3-methyl-2-(3-pyridinyl)-1H-indol-octanoic acid, (E)-7-phenyl-7-(3-pyridyl)-6-heptenoic acid, dazoxiben, (±)-6-(1-imidazolylmethyl)-5,6,7,8-tetrahydronaphthalene-2-carboxylic acid, furegrelate, ozagrel, pirmagrel, 4-[α-hydroxy-5-(1-imidazolyl)-2-methylbenzyl]-3,5-dimethylbenzoic acid, dazmegrel, midazogrel, (E)-1-[3-(phenylmethoxy)-1-octenyl]-1H-imidazole, daltroban, sulotroban,7-[2α,4α-(dimethylmethano)-6β-(2-cyclopentyl-2β-hydroxyacetamido)-1α-cyclohexyl]-5(Z)-heptanoic acid, sodium (E)-11-[2-(5,6-dimethyl-1-benzimidazolyl)ethylidene]-6,11-dihydrobenz[b,e]oxepin-2-carboxylate, vapiprost, ticlopidine, clopidgrel, beraprost, iloprost, 5-{(1R,6S,7S,8R)-8-hydroxy-7-[(3S)-3-hydroxy-4,4-dimethyl-1,6-nonadiynyl]-cis-bicyclo[4,3,0]non-2-en-3-yl]}-3-oxapentanoic acid, methyl 5-{(1S,5S,6R,7R)-7-hydroxy-6-[(E)-(S)-3-hydroxy-1-octenyl]bicyclo[3,3,0]oct-2-en-3-yl]pentanoate and the like.

Further, examples of the thrombin inhibitor include argatroban, (2S)-2-[4-[[(3S)-1-acetoimidoyl-3-pyrrolidinyl]oxy]phenyl]-3-(7-aminidino-2-naphthyl)-propenoic acid and the like.

Examples of the other antithrombotic drug include warfarin, pentoxifylline, verapamil, diltiazem, (-)-cis-3-acetoxy-5-[2-(dimethylamino)ethyl]-2,3-dihydro-8-methyl -2-(4-methylphenyl)-1,5-dibenzodiazepine-4-(5H)-one and the like.

In the present invention, it is particularly preferred to use cilostazol, dipyridamole, aspirin, satigrel, etc., among these antithrombotic drugs.

The amount of the antithrombotic drug is 0.01 to 150 parts by weight, preferably 0.1 to 100 parts by weight, based on 100 parts by weight of the polymer material. When the amount of the antithrombotic drug exceeds the above range, molding properties become inferior. If the molding can be conducted, physical properties are deteriorated and, therefore, it is not suitable for practical use. On the other hand, when the amount of the antithrombotic drug is smaller than the above range, it becomes difficult to control the release of the antithrombotic drug. Therefore, it becomes difficult to inhibit blood coagulation, efficiently, which results in no addition effect of the antithrombotic drug. Further, it becomes possible to control the release amount of the antithrombotic drug by varying the amount of the antithrombotic drug within the above range.

Examples of the shape of the antithrombotic drug releasing member containing the antithrombotic drug include film, filament, granule, tube and the like. It is possible to control releasing properties of the antithrombotic drug by changing the thickness and surface area in case of the film, size and length in case of the filament, shape of particles, surface area and particle size in case of the granule, and thickness and surface area in case of the tube, respectively. It is also possible to increase releasing properties by subjecting the surface of the antithrombotic drug releasing member to embossing. Furthermore, it is also possible to control releasing properties more precisely by using two or more sorts of shapes and specifications of antithrombotic drug releasing members in combination.

Typical production process of the antithrombotic drug releasing member of the present invention includes a solution method and a melting method. In the solution method, the polymer material and antithrombotic drug are uniformly dissolved in a solvent and then the solvent is removed to give a releasing member. Examples of the solvent include dimethylformamide, dimethylacetamide, cyclohexanone, tetrahydrofuran, dioxane, chloroform, 1,2-dichloroethane, methylene chloride, methyl ethyl ketone, acetone, ethanol, isopropanol, methanol, 2,2,2-trifluoroethanol and 1,1,1,3,3,3-hexafluoro-2-propanol, and a mixed solvent of two or more sorts of them. Among them, tetrahydrofuran is preferable to polyvinyl chloride, 1,1,1,3,3,3-hexafluoro-2-propanol is preferable to the ethylene-vinyl alcohol copolymer, and chloroform, methylene chloride and 1,2-dichloroethane are preferable to polycarbonate, polyacrylate and acetylated cellulose, respectively, because the above solvents have high solvency and can be easily distilled.

It is preferred that the polymer material to be used is sufficiently washed by a method such as Soxhlet extraction in advance to remove impurities in the polymer material. Further, it is preferred that it is sufficiently dried to remove water in the polymer material.

The molding can be conducted by casting a solution wherein the above respective components are dissolved in a solvent on a glass plate, or extruding into a tubular extrudate or applying on the other structure, followed by removing the solvent. Thereby, the polymer material can be made into a film-like or tubular form, or coated. The solvent can be removed by air-drying, drying with heating under reduced pressure, phase transition due to a coagulating solution and the like. Examples of the coagulating solution include poor solvents of the polymer material to be used, e.g. water, alcohols such as methanol, ethanol, butanol, etc., ketones such as acetone, etc. In this case, it is necessary to prevent the antitheromogenic drug from dissolving in the coagulating solution during coagulation of the polymer material in every way. Therefore, it is preferred to mix a solvent for reducing a solvency of the antithrombotic drug (e.g. glycerin, etc.) with the poor solvent of the polymer material to coagulate the polymer material and the antithrombotic drug contained therein, simultaneously.

When molding the tubular antithrombotic drug releasing member by the solution method, a solution wherein the above respective components are dissolved in a solvent is applied on the surface of a suitable stem and then dried to form a tube, which is stripped from the stem. Otherwise, the above solution is applied on the surface of the stem, which is dipped in a coagulating solution to coagulate the polymer on the surface of the stem to give a tube and, thereafter, the resulting tube is stripped from the stem. The tubular device can also be produced by drying after the solution was extruded into a hollow form in the coagulating solution.

When preparing a film-like antithrombotic drug releasing member by the solution method, for example, there can be used a method of molding into a film such as a method comprising casting a solution on a glass plate and then drying to remove the solvent, a method comprising coating a solution directly on a woven fabric, knitted web, non-woven fabric, etc. and then drying to remove the solvent and the like. Further, the film-like material can also be produced by coating onto a ready-made film according to dipping method, spraying method and the like. The film molded using any one of polymer materials can be further coated with the other polymer material to produce a multi-layer structure film.

In the solution method, the rate of the antithrombotic drug to be released from a molded article can be controlled by varying the amount of the antithrombotic drug contained in the polymer material, kind of the polymer material, method of removing the solvent (e.g. a method of drying under normal or reduced pressure, or a method of coagulating using a coagulating solution) and the like. Particularly, when using plasticized polyvinyl chloride as polyvinyl chloride, the release rate can also be controlled by the blending formulation of plasticizers, stabilizers, secondary plasticizers, lubricants and the like. In case of the coating, the release amount can be controlled more precisely by repeating coating plural times and varying conditions such as amount of the antithrombotic drug, kind of the polymer material, etc.

The solution methods described above are particularly useful when the antithrombotic drug to be used is thermally unstable.

On the other hand, in case of the melting method, the polymer material and antithrombotic drug are molten and then molded to obtain an antithrombotic drug releasing member. The melting must be conducted so that the antithrombotic drug is uniformly dispersed in the polymer material without causing decomposition of the antithrombotic drug. Therefore, the suitable antithrombotic drug and polymer material may be selected so that the polymer material is molten at a temperature lower than a decomposition temperature of the antithrombotic drug. Further, if necessary, oxidative degradation of the antithrombotic drug and polymer material can be prevented if melting and molding operations are conducted in inert gas atmosphere. It is preferred to remove water in the polymer material to be used as much as possible in view of stability in molding as well as that in antithrombotic drug and polymer material.

Various molding methods can be used for the melting method, for example, a tubular, sheet-like or filament-like molded article and a molded article of a complicated structure can be molded by an extrusion molding and injection molding, respectively.

The release of the antithrombotic drug from the molded article in the melting method can also be controlled by varying the amount of the antithrombotic drug in the polymer material, kind of the polymer material to be used and the like. Particularly, when using polyvinyl chloride, it is possible to control the release of the antithrombotic drug by the blending formulation of additives such as plasticizers, stabilizers, secondary plasticizers, lubricants and the like, similar to the solution method. By conducting the multi-layer (multi-color) molding and varying the amount or kind of the antithrombotic drug in the respective layers (parts), physical properties required for the antithrombotic drug releasing member can be obtained and, at the same time, the antithrombotic activity can be developed only at the desired part and the release can be controlled more precisely.

The vessel for the antithrombotic drug releasing member of the present invention can be obtained by molding a suitable polymer material. The usable polymer material may be any polymer material causing no sanitary problem, and examples thereof include polyethylene, polypropylene, polyvinyl chloride, poly 4-methyl-1-pentene, polycarbonate and the like. Further, the molding method can be optionally selected from methods which correspond to a normal molding method of plastic molded articles, such as blow molding method, injection molding method and the like.

The amount of the antithrombotic drug releasing member to be contained in the vessel can be adjusted depending upon the release amount (rate) of the antithrombotic drug, condition of a patient, etc., and is normally within a range of 1 to 1000 mg, preferably 10 to 500 mg as antithrombotic drug.

Further, the vessel can also be molded using the antithrombotic drug releasing member itself. In this case, the antithrombotic drug is released from the inner surface of the vessel even if the vessel contains no antithrombotic drug releasing member. Accordingly, the vessel constitutes a part of a blood circuit in this case. Further, the inner surface of the above normal vessel obtained by molding the polymer material may be coated with the antithrombotic drug releasing member, and the vessel may also contain the antithrombotic drug releasing member. In these cases, the amount of the antithrombotic drug is the same as that of the antithrombotic drug for the polymer material.

Further, it is preferred that the vessel in the present invention is connected to the blood circuit, easily. Accordingly, the vessel can be connected to any position by changing the shape of the connector section formed on a blood inlet and a blood outlet, which are respectively provided on the vessel.

Examples of the extracorporeal circulation to which the present invention can be applied include blood purification method such as hemodialysis, hemofiltration, hemodiafiltration, plasmapheresis, etc., or extracorporeal circulation at the time of surgery such as open heart surgery using an artificial heart-lung, liver surgery, etc. The method of the present invention whose anticoagulant mechanism is different from conventional ones, is useful particularly for blood clotting, residual blood deterioration in dialysis efficiency due to platelet deposition onto a separation membrane under blood purification even when heparin is in use. Further, when it is used for the extracorporeal circulation in the open heart surgery and cilostazol, dipyridamole, etc. are used as the antithrombotic drug, the drug released has not only anticoagulation action but also vasodilation action so that circulatory failure caused by controlled shock of the peripheral tissue due to extracorporeal circulation can be improved.

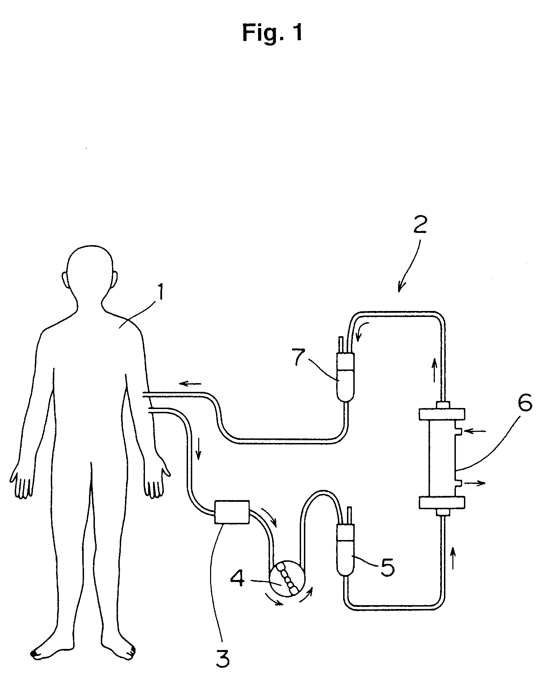

Fig. **1** is an explanatory view illustrating one embodiment of the method for anticoagulation of the present invention applied to an extracorporeal circulation system for hemodialysis. In the extracorporeal circulation system shown in Fig. **1**, an antithrombotic drug releasing device **3**, a blood pump **4** (roller pump), a drip chamber **5**, a dialyzer **6** and a drip chamber **7** are connected in turn to a blood circuit **2** led outside the body from a patient **1** along with a flow direction of blood.

Blood drawn from the body of the patient **1** by a blood pump **4** is firstly passed through an antithrombotic drug releasing device **3**. In that case, the antithrombotic drug having the concentration enough to inhibit blood coagulation is released into blood. In this state, the flow rate of blood is adjusted by passing through a blood pump **4** and a drip chamber **5**, and blood is passed to a dialyzer **6** and dialyzed. Then, blood is returned to the body of the patient **1** via the drip chamber **7**.

Since a predetermined amount of the antithrombotic drug, which is slightly soluble in water, is forced to release into the blood circuit **2** from the antithrombotic drug releasing device **3**, as described above, the concentration of the antithrombotic drug in the body of the patient **1** can be maintained at a low level in comparison with a conventional systemic oral administration which requires a high dose until the concentration reaches an effective concentration in the blood circuit even if the antithrombotic drug flows into the body of the patient **1** from the blood circuit **2**. Therefore, the method of the present invention is effective to avoid the risk of side effects.

The method of the present invention can be applied to various blood circuits used in hemofiltration, hemodiafiltration, artificial heart-lung, etc., in addition to hemodialysis in a similar way.

### FIELD OF THE INDUSTRIAL APPLICABILITY

As described above, the present invention has such an effect that an antithrombotic drug can be continuously released from an antithrombotic drug releasing member wherein the antithrombotic drug which is slightly soluble in water is dispersed in a specific polymer material so that blood coagulation in a blood circuit can be inhibited by bringing the antithrombotic drug releasing member into contact with blood, directly, in the blood circuit of an extracorporeal circulation system.

### EXAMPLES

The following Examples further illustrate the present invention in detail but are not to be construed to limit the scope thereof.

### Example 1

### (1) Preparation of antithrombotic drug releasing member (film) wherein cilostazol is uniformly dispersed in an ethylene-vinyl alcohol copolymer by means of a melting method

An ethylene-vinyl alcohol copolymer (Soarnol AT4403, manufactured by the Nippon synthetic Chemical Industry Co., Ltd.) was pulverized using an analytical pulverizer (Model R-8, manufactured by Nippon Rikagaku Kikai Co., Ltd.) and screened to collect powder of 125 to 50 µm in particle size. Cilostazol was dry blended with the resulting powder, and the blend was pressed at 180°C for 2 minutes using a compact type test press (manufactured by Toyo Seiki Co., Ltd.) to prepare a film of 100 µm in thickness. The amount of cilostazol contained in this film was 10 % by weight.

### (2) Antithrombotic drug dissolution test

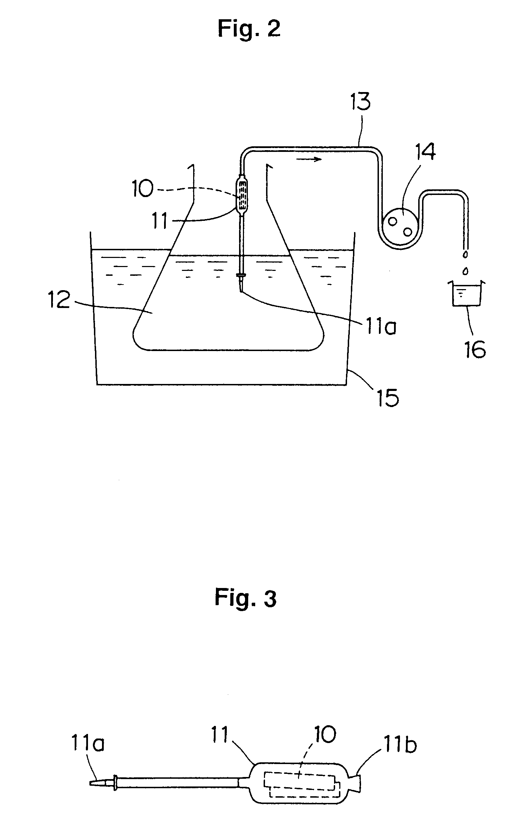

The resulting film (500 mg) was put in a vessel made of polyvinyl chloride and a physiological saline solution (pH 7.4) wherein serum albumin was added in the concentration of 5 % by volume in place of blood was used as a dissolution solution to evaluate dissolution properties. Referring to an assembled device shown in Fig. **2**, a tip section **11a** of a vessel **11** containing a predetermined amount of a film **10** is put in a serum albumin-containing physiological saline solution **12** maintained at 37°C in a thermostatic water bath **15**. A transfer tube **13** made of polyvinyl chloride was connected with a rear end outlet **11b** of the vessel **11**, and the physiological saline solution **12** was pumped up by rotating a roller pomp **14** to pass it through the vessel **11**, then to discharge through the transfer tube **13**. Fig. **1** is a schematic diagram illustrating a whole hemodialyzer reduced to a scale of 1/2.5 the natural size. Fig. **3** illustrates the vessel **11** containing the film **10** used in Fig. **2**.

The serum albumin-containing saline solution **12** discharged from the rear end of the transfer tube **13** was collected as a sample in a receptacle **16** every 5 minutes. The concentration of cilostazol in the serum albumin-containing physiological saline solution **12** was determined. As a result, the concentration of cilostazol in the serum albumin-containing physiological saline solution exceeded an effective concentration (1.1 µg/ml) required to exhibit a sufficient anticoagulant action, at all points of time from the beginning of the test to the time after 5 hours.

### Example 2

### (1) Preparation of antithrombotic drug releasing member (film) wherein cilostazol is uniformly dispersed in an ethylene-vinyl alcohol copolymer by means of a solution method

An ethylene-vinyl alcohol copolymer (Soarnol 3825N, manufactured by the Nippon Synthetic Chemical Industry Co., Ltd.) (720 mg) and cilostazol (80 mg) were dissolved in 20 ml of 1,1,1,3,3,3-hexafluoro-2-propanol and the mixture was casted/applied on a glass plate, followed by drying at room temperature and additional vacuum drying at 40°C with a vacuum oven to give a transparent film of about 50 µm in thickness. The amount of cilostazol contained in this film was 10 % by weight.

### (2) Antithrombotic drug dissolution test

According to the same manner as that described in Example 1 (2), the dissolution properties were evaluated using the resulting film. As a result, the concentration of cilostazol in the serum albumin-containing physiological saline solution exceeded an effective concentration (1.1 µg/ml) required to exhibit a sufficient anticoagulant action, at all points of time from the beginning of the test to the time after 5 hours.

### Example 3

### (1) Preparation of antithrombotic drug releasing member (film) wherein cilostazol is uniformly dispersed in triacetyl cellulose by means of a solution method

Triacetyl cellulose (0.9 g, manufactured by Aldrich Chemical Co.) and cilostazol (0.1 g) were dissolved in 25 ml of chloroform and the solution was casted on a glass plate, followed by drying at room temperature for 12 hours and additional vacuum drying at 40°C with a vacuum oven to give a transparent film of about 50 µm in thickness. The amount of cilostazol contained in this film was 10 % by weight.

### (2) Antithrombotic drug dissolution test

According to the same manner as that described in Example 1 (2), the dissolution properties were evaluated using the resulting film. As a result, the concentration of cilostazol in the serum albumin-containing physiological saline solution exceeded an effective concentration (1.1 µg/ml) required to exhibit a sufficient anticoagulant action, at all points of time from the beginning of the test to the time after 5 hours.

### Example 4

### (1) Preparation of antithrombotic drug releasing member (film) wherein cilostazol is uniformly dispersed in an ethylene-vinyl alcohol copolymer by means of a melting method

An ethylene-vinyl alcohol copolymer (Soarnol AT4403, manufactured by the Nippon Synthetic Chemical Industry Co., Ltd.) (90 g) and cilostazol (10 g, manufactured by Otsuka Pharmaceutical Co., Ltd.) were mixed and extruded at 180°C under a nitrogen atmosphere by a mixing extruder (MAX MIXING EXTRUDER, Model CS194A MAX, manufactured by CSI Co.), using a strand die. Immediately after that, the extrudate was hot-pressed using a compact type test press (manufactured by Toyo Seiki Co., Ltd.) to give an ethylene-vinyl alcohol copolymer film of 100 µm in thickness. The amount of cilostazol contained in this film was 10 % by weight.

### (2) Antithrombotic drug dissolution test

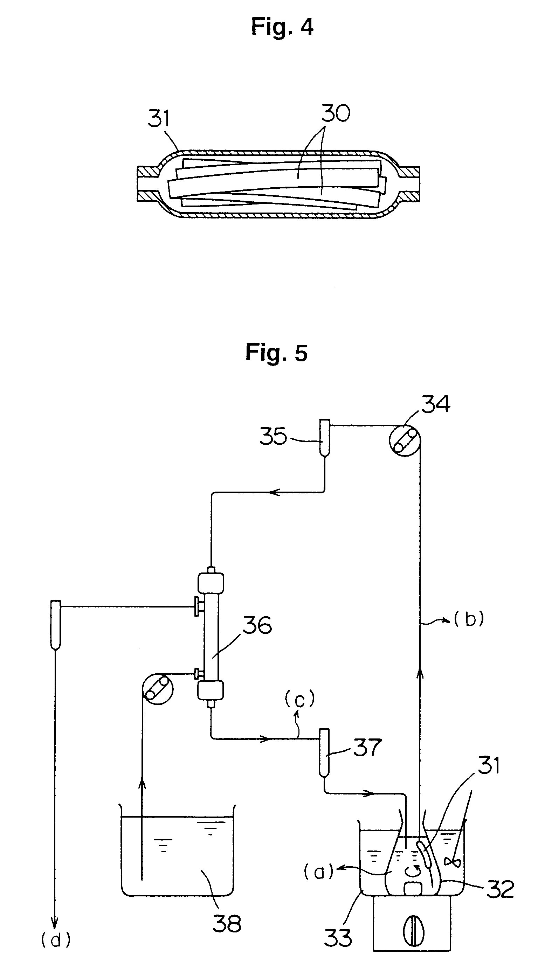

Firstly, as shown in Fig. **4**, a film **30** as the antithrombotic drug releasing member obtained in the above item (1) was inserted in a vessel **31** made of polyvinyl chloride (capacity of film 30 to be contained: 500 mg). Then, the dissolution properties of the antithrombotic drug were evaluated using a hemodialysis model shown in Fig. **5**. Referring to the dialysis model shown in Fig. **5**, a physiological saline solution (pH 7.4) wherein serum albumin was added in the concentration of 5 % by volume in place of blood was charged in a flask **32** and maintained at 37°C in a thermostatic water bath **33**. Further, this physiological saline solution was passed through the vessel **31** and flowed through a roller pump **34** and a drip chamber **35** to a dialyzer **36** (Model AM-SD-06M, manufactured by Asahi Medical Co., Ltd.), where the physiological saline solution was dialyzed. Thereafter, the dialyzed solution was returned to the flask **32** via another drip chamber **37**, which forms a circulation circuit (circulation rate of the physiological saline solution: **40** ml/minute). Further, a dialysis solution **38** (flow rate: 200 ml/minute) maintained at 37°C was passed through the dialyzer **36**.

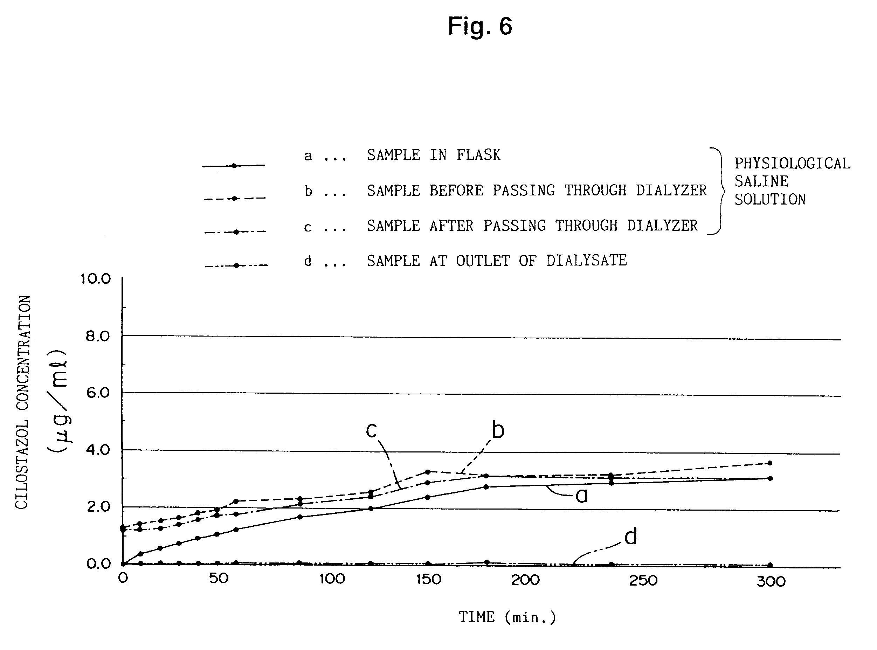

In order to examine dissolution properties, the followings were collected as samples:
**a**...a physiological saline solution in the flask **32**;
**b**...a physiological saline solution flowing before passing through the dialyzer **36**;
**c**...a physiological saline solution flowing after passing through the dialyzer **36**; and
**d**...a dialysis solution at an outlet after passing through the dialyzer **36**. The sample (2 ml) was collected every 10 minutes, 30 minutes and 1 hour, from the beginning of the circulation to the time after 1 hour, 1 to 3 hours and 3 to 5 hours, respectively. The concentration of cilostazol in each sample was determined, respectively. The results are shown in Fig. **6**.

As is apparent from Fig. **6**, a high anticoagulant activity is exhibited even in the blood circuit at the time of actual dialysis, because cilostazol is continuously dissolved from the film **30** in the vessel **31** in the concentration exceeding the effective concentration (1.1 µg/ml) when the physiological saline solution containing serum albumin as blood is extracorporeally circulated.

### Example 5

### (Preparation of extracorporeal circulation system for hemodialysis, hemofiltration and hemodiafiltration)

### (1) Production of vessel

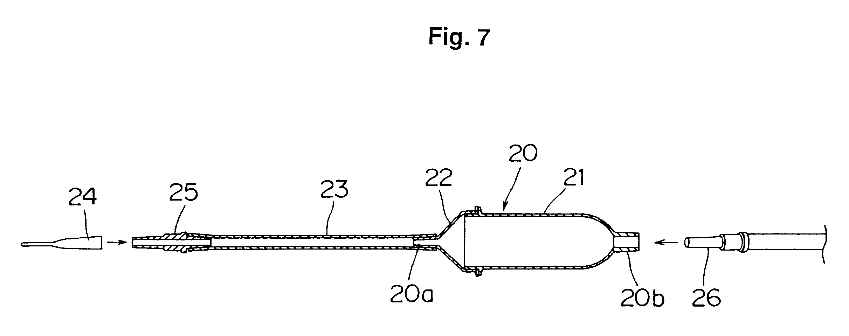

A vessel **20** shown in Fig. **7** was prepared. This vessel **20** comprises a body **21** and a lid section which are molded from polypropylene using an injection molding machine. A blood inlet **20a** of this vessel **20** was connected with a tube **23** made of polyvinyl chloride, and a tip of this tube **23** was connected with a connector **25** made of polypropylene, which can be quickly connected with a cannula **24** (fistula) for blood access at the time dialysis. Further, a blood outlet **20b** of the vessel **20** can be quickly connected with a tip section **26** of a normal blood circuit.

### (2) Preparation of antithrombotic drug releasing member (film) wherein cilostazol is uniformly dispersed in an ethylene-vinyl alcohol copolymer

An ethylene-vinyl alcohol copolymer (EVAL ES-G, 110A, manufactured by KURARAY CO., LTD.) (180 g) and cilostazol (20 g) were extruded by a mixing extruder (manufactured by CSI Co.), using a film die to prepare a film of about 100 µm in thickness. This film was subjected to embossing and provided a pleated structure using a heat-roll, thereby affording a cilostazol-containing ethylene-vinyl alcohol copolymer film. The amount of cilostazol contained in this film was 10 % by weight.

### (3) Preparation of extracorporeal circulation system of blood

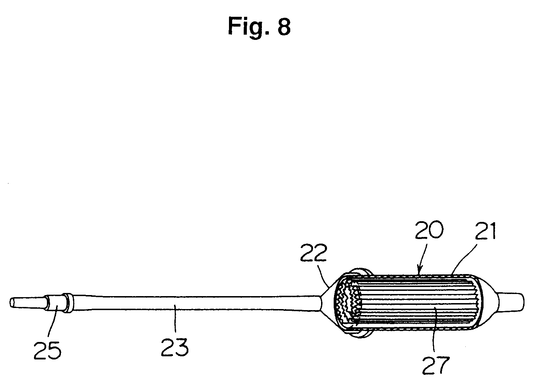

As shown in Fig. **8**, 1 g (corresponding to 100 mg of cilostazol) of the film **27** obtained in the above item (2) was put in a vessel **20** and, after sealing a body **21** of the vessel **20** to a lid **22** integrally by an ultrasonic welding, the whole was sterilized and used after connecting with a blood circulation system as shown in Fig. **1**. In that case, the amount of the film **27** can be adjusted depending upon the condition of the patient. Further, a plurality of vessels **20** can be used to connect them in series or parallel.

### Example 6

### (Preparation of blood circulation system for artificial heart-lung)

### (1) Production of vessel

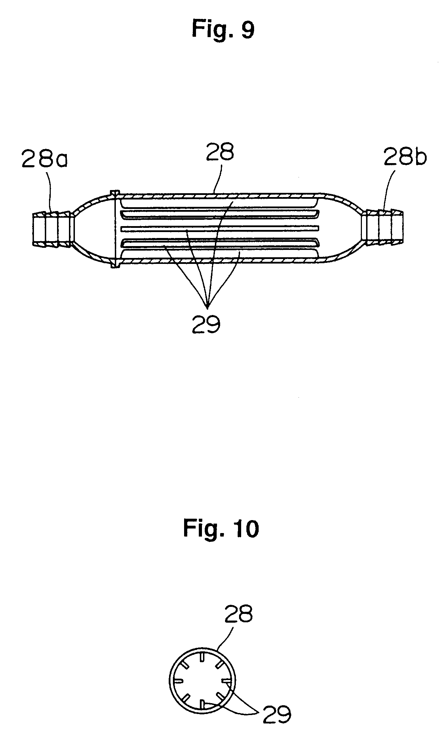

A vessel **28** shown in Fig. **9** or **10** was prepared. This vessel **28** is provided with a blood inlet **28a** and a blood outlet **28b** at both ends and is provided protrudingly with a plurality of ribs **29** in the inner surface of the body, which was molded from polypropylene using an injection molding machine. The ribs **29** serve to prevent the antithrombotic drug releasing member contained in the vessel **28** from adhering to the wall inner surface of the vessel, which results in deterioration of the drug releasing effect.

### (2) Preparation of antithrombotic drug releasing member wherein cilostazol is uniformly dispersed in an ethylene-vinyl alcohol copolymer

According to the same manner as that described in Example 4 (2), a film as the titled antithrombotic drug releasing member was prepared.

### (3) Preparation of extracorporeal circulation system of blood

The film obtained in the above item (2) (1 g, corresponding to 100 mg of cilostazol) was put in the vessel **28** obtained in the above item (1) and, after ultrasonic welding, the whole was sterilized. The sterilized vessel containing the film was incorporated into a venous return cannulae from the body in the blood circulation system for artificial heart-lung so that blood may be passed through the vessel **28** to an artificial lung using a blood pump. Further, blood supplied with oxygen in the artificial lung leaves out of the artificial lung and returns to the heart in the body through an arterial perfusion cannulae. In that case, the amount of the antithrombotic drug releasing film **27** can be adjusted according to the condition of a patient. Further, a plurality of vessels **28** can be used to connect them in series or parallel.

## Claims

1. A method for anticoagulation in the extracorporeal circulation of blood, which comprises bringing an antithrombotic drug releasing member, which is formed by dispersing an antithrombotic drug in a polymer material, into contact with blood at the time of the extracorporeal circulation of blood, said antithrombotic drug being slightly soluble in water.

2. The method for anticoagulation according to claim 1, wherein the antithrombotic drug releasing member contacts with blood at a section to initiate the extracorporeal circulation, which is close to the human body, or the vicinity thereof.

3. The method for anticoagulation according to claim 1 or 2, wherein the antithrombotic drug is cilostazol.

4. The method for anticoagulation according to claim 3, wherein the polymer material is ethylene-vinyl alcohol copolymer.

5. An antithrombotic drug releasing device to be installed in a blood circuit for extracorporeal circulation, comprising a vessel provided with a blood inlet and a blood outlet, and an antithrombotic drug releasing member contained in the vessel, said antithrombotic drug releasing member being formed by dispersing an antithrombotic drug in a polymer material.

6. An antithrombotic drug releasing device to be installed in a blood circuit for extracorporeal circulation, comprising a vessel provided with a blood inlet and a blood outle, and at least an inner wall surface of said vessel comprising an antithrombotic drug releasing member wherein an antithrombotic drug is dispersed in a polymer material.

7. The antithrombotic drug releasing device according to claim 5 or 6, wherein the antithrombotic drug is cilostazol.

8. The antithrombotic drug releasing device according to claim 7, wherein the polymer material is ethylene-vinyl alcohol copolymer.
